(19) Europäisches Patentamt
European Patent Office
Office européen des brevets

(11) Numéro de publication: **0 196 253**
**B1**

# (12) FASCICULE DE BREVET EUROPEEN

(45) Date de publication du fascicule du brevet: **08.08.90**

(21) Numéro de dépôt: **86400528.5**

(22) Date de dépôt: **12.03.86**

(51) Int. Cl.$^5$: **B 01 J 31/18,** C 07 C 45/00, C 25 B 3/12

## (54) Système catalytique, son procédé de préparation et son application à la fabrication d'aldéhydes.

(30) Priorité: **13.03.85 FR 8503670**

(43) Date de publication de la demande:
**01.10.86 Bulletin 86/40**

(45) Mention de la délivrance du brevet:
**08.08.90 Bulletin 90/32**

(84) Etats contractants désignés:
**AT BE CH DE FR GB IT LI LU NL SE**

(56) Documents cités:
**FR-A-2 080 556**
**FR-A-2 550 201**
**GB-A-1 182 651**
**US-A-4 358 621**
**US-A-4 370 258**

**CHEMICAL ABSTRACTS, vol. 101, no.16, 15 octobre 1984, page 559, résumé no. 139669x, Columbus, Ohio, US; J. GROBE et al.: "Electrochemical syntheses, XI. Catalytic application of electrochemical systems: hydroformylation reactions", & Z. NATURFORSCH., B: ANORG. CHEM. ORG. CHEM. 1984, 39B(7), 962-7**

(73) Titulaire: **EXXON CHEMICAL PATENTS INC.**
**1900 East Linden Avenue**
**Linden New Jersey 07036 (US)**

(72) Inventeur: **Mortreux, André**
**17 rue Gambetta**
**F-59510 Hem (FR)**
Inventeur: **Petit, Françis**
**27 Allée de la Clairière**
**F-59650 Villeneuve d'Ascq (FR)**
Inventeur: **Mutez, Sylvain**
**2/27 Avenue Kennedy**
**F-59200 Tourcoing (FR)**
Inventeur: **Paumard, Eric**
**58 rue Jules Fostier**
**F-59790 Ronchin (FR)**

(74) Mandataire: **Bawden, Peter Charles et al**
**EXXON CHEMICAL LIMITED EXXON CHEMICAL TECHNOLOGY CENTRE PO Box 1**
**Abingdon Oxfordshire OX13 6BB (GB)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 196 253 B1**

**Description**

La présente invention concerne un système catalytique, un procédé pour sa préparation et son application à la fabrication d'aldéhydes par hydroformylation de composés organiques à insaturation éthylénique.

Le but de la présente invention consiste à obtenir, par hydroformylation des oléfines, un mélange d'aldehydes normaux et ramifiés dans lequel la proportion d'aldéhydes normaux soit la plus élevée possible.

Un premier objet de la présente invention consiste en un système catalytique caractérisé en ce qu'il comprend:

au moins un complexe de platine de formule $LPtX_2$ dans laquelle L est un composé organique comportant au moins deux atomes de phosphore capables de coordiner le platine et X est un atome d'halogène, et

au moins une combinaison complexe formée entre l'étain et un carbonate d'alcène, le groupe alcène ayant de préférence de 2 à 6 atomes de carbone.

Dans le système catalytique selon la présente invention, le complexe de platine et la combinaison complexe à base d'étain sont de préférence présents en proportions respectives telles que le rapport atomique Sn/Pt soit compris entre 0,2 et 5.

Le système catalytique selon l'invention comprend comme premier composant au moins un complexe de platine de formule $LPtX_2$ dans laquelle X peut être choisi de manière quelconque parmi le fluor, le chlore, le brome et l'iode. Le composé organique de phosphore L peut être par exemple:

un bis(diphénylphosphino)alcane de formule

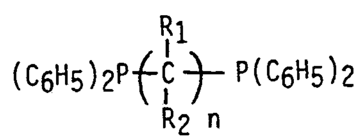 (I)

dans laquelle $R_1$ et $R_2$, identiques ou différents, sont choisis parmi l'atome d'hydrogène et les radicaux hydrocarbonés aliphatiques, ceux-ci étant le cas échéant fonctionnalisés et/ou liés l'un à l'autre, et n est supérieur ou égal à 4.

Un aminophosphine-phosphite de formule

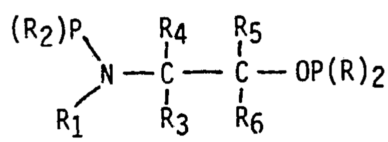 (II)

dans laquelle:

R est un radical hydrocarboné aliphatique, cycloaliphatique ou aromatique,

$R_1$ est choisi parmi l'atome d'hydrogène et les radicaux hydrocarbonés,

$R_3$ et $R_4$, nécessairement différents l'un de l'autre, sont choisis parmi l'atome d'hydrogène et les radicaux hydrocarbonés éventuellement porteurs d'au moins une fonction choisie parmi les fonctions alcool, thiol, thioether, amine, imine, acide, ester, amide et ether, et

$R_5$ et $R_6$ sont choisis parmi l'atome d'hydrogène et les radicaux hydrocarbonés éventuellement fonctionnalisés.

Le (1S,2S)(+)trans-1,2-bis(diphénylphosphinométhyl)-cyclohexane, le 1,4-bis(diphénylphosphino)butane et l'isopropylidène dihydroxy-2,3-bis-(diphénylphosphino)-1,4-butane constituent des exemples de composés organiques de phosphore de formule (I) pouvant être utilisés dans le cadre de la présente invention. D'autre part certains composés de formule (II) et leur mode de préparation ont été décrits dans la demande de brevet français n° 2.550.201.

Le système catalytique selon l'invention comprend en outre au moins une combinaison complexe formée entre l'étain et un carbonate d'alcène, tel que le carbonate de propylène, le carbonate d'éthylène, le carbonate de 1,2-butylène et le carbonate de 1,2-hexylène.

La dite combinaison complexe peut être formée par réduction d'un solvant électrochimique comportant au moins un carbonate d'alcène dans une cellule électrochimique comprenant une anode en étain et une cathode en platine en portant la cathode à un potentiel inférieur ou égal à — 0.2 V, et en maintenant constant ce potentiel pendant une durée suffisante pour assurer la production d'une quantité d'électricité supérieure ou egale à 0,5 Faraday par atome-gramme de platine. Dans ce cas, la cellule électrochimique peut comprendre en outre une électrode de référence choisie parmi des électrodes connues telles que notamment Ag/AgCl, $Ag/Ag^+$, $Hg/Hg_2Cl_2$ (calomel), le potentiel de la cathode étant alors calculé par rapport à la dite électrode de référence. Selon un mode de réalisation de la présente invention, la réduction du solvant électrochimique peut être effectuée en présence d'une faible quantité d'un sel conducteur soluble dans le solvant électrochimique tel que, par exemple, l'hexafluorophosphate de tétra-n-butyle ammonium. La présence de ce sel conducteur permet avantageusement d'accélérer la réduction du

solvant, en particulier à température modérée. La phase de réduction électrochimique selon l'invention est généralement effectuée à une température comprise entre 10°C et 70°C et en maintenant la cellule électrochimique sous atomosphère d'un gaz inerte tel que l'azote. Le solant électrochimique utilisé dans le cadre de la présente invention comprend nécessairement au moins un carbonate d'alcéne tel que défini précédemment. Il peut comprendre en outre, en mélange avec ce dernier, un autre solvant tel que par exemple un hydrocarbure aromatique.

Un second object de la présente invention consiste en un procédé de préparation d'un système catalytique tel que décrit précédemment. Un tel procédé consiste à fair réagir au moins une combinaison complexe formée entre l'étain et le carbonate d'alcène avec au moins un complexe de platine de formule $LPtX_2$ dans au moins un solvant dudit complexe, à une température comprise entre 10°C et la température d'ébullition dudit solvant. Parmi les solvants du complexe de platine de formule $LPtX_2$ on peut citer notamment les hydrocarbures aromatiques (tels que par exemple le benzène, le toluène et les xylènes) et les carbonates d'alcène, notamment ceux dont le groupe alcène a de 2 à 6 atomes de carbone. Pour la mise en oeuvre du procédé selon l'invention, on préférera utiliser un solvant comprenant au moins 25% en volume de carbonate d'alcène. La durée de la réaction entre la combinaison à base d'étain et le complexe de platine peut varier, selon les règles usuelles bien connues de l'homme de l'art, en fonction de la température de réaction choisie et de la concentration des espèces réactives dans le solvant. A titre d'exemple cutte durée n'est généralement pas supérieure à 20 minutes losque la température de réaction est égale à 80°C.

Plusieurs modes de réalisation peuvent être envisagés dans le cadre du procédé selon la présente invention. Un premier mode de réalisation consiste à former une combinaison complexe d'étain et d'un carbonate d'alcène, par exemple par une méthode électrochimique telle que décrite précédement, puis à introduire le complexe de platine dans le milieu dans lequel la combinaison complexe d'étain a été formée, ledit milieu comprenant déjà le solvant nécessaire à la réaction. Un second mode de réalisation consiste à former une combinaison complexe d'étain et d'un carbonate d'alcène, par exemple par une méthode électrochimique telle que décrite précédemment, à isoler sous forme de poudre ladite combinaison complexe du milieu dans lequel elle a été formée (par exemple en filtrant, lavant et sèchant le précipité qui se forme dans la cellule électrochimique), puis à introduire ladite poudre dans une solution du complexe de platine dans le solvant nécessaire à la réaction. Enfin un troisième mode de réalisation consiste à former la combinaison complexe d'étain et d'un carbonate d'alcène en présence du solvant et du complexe de platine, par exemple par une méthode électrochimique telle que décrite précédemment.

Quelque soit le mode de réalisation choisi pour le procédé selon l'invention, il est souhaitable que la concentration du complexe de platine dans le solvant de la réaction soit comprise entre 0,01 et 0,2 mole par litre.

Un troisième object de la présente invention consiste en l'application du système catalytique décrit précédemment à la fabrication d'aldéhydes par hydroformylation d'un composé organique à insaturation éthylénique, caractérisé en ce que l'on fait réagir, à une température comprise entre 10°C et 300°C et sous une pression comprise entre 1 et 350 bars, ledit composé organique avec un mélange de monoxyde de carbone et d'hydrogène en présence d'une quantité efficace dudit système catalytique. Une quantité efficace de système catalytique est généralement telle que le rapport molaire du composé organique à insaturation éthylènique au platine soit compris entre 100 et 10.000.

Le rapport molaire $CO/H_2$ dans le mélange de monoxyde de carbone et d'hydrogène intervenant dans la réaction d'hydroformylation selon l'invention et généralement compris entre 0,5 et 2.

Parmi les composés organiques à insaturation éthylénique pouvant être soumis à la réaction d'hydroformylation selon l'invention, on peut citer notamment:

les oléfines ayant de 2 à 12 atomes de carbone, telles que notamment le propylène, le butène-1, l'hexène-1, l'oxtène-1, etc.

les composés vinyliques aromatiques tels que le styrène.

les diènes tels que par exemple le vinyl-4-cyclohexène.

La durée de la réaction d'hydroformylation selon l'invention est généralement comprise entre 0,5 et 30 heures.

Les exemples ci-après sont donnés à titre illustratif et non limitatif de la présente invention:

## Exemples 1 à 4

## Préparation de systèmes catalytiques

Dans une cellule électrochimique en verre, on introduit sous azote 60 mg de complexe $LPtCl_2$, le ligand L'étant l'isopropylidène dihydroxy-2,3-bis(diphénylphosphino)-1,4 butane, puis le solvant constitué de 25 $cm^3$ d'un mélange benzène et de carbonate de d'alcène. Après dissolution complète du catalyseur, on plonge dans le solvant la cathode (constituée d'un panier de platine) et l'anode (constituée d'un cylindre d'étain), puis l'électrode de référence ($Ag/AgCl/N(C_2H_5)_4Cl$ 0.1 M dans le carbonate d'alcène). La température étant égale à 20°C, on fixe le potentiel de réduction à $-1.85V$ et la quantité de courant passant dans le circuit est mesurée à l'aide d'un coulomètre. La quantité d'étain nécessaire à la réaction est mesurée par pesée de l'anode avant et après la réduction (la masse obtenue par pesée est généralement indentique à celle déterminée à partir de la quantité de coulombs passant dans le circuit). Dans ces conditions le rapport atomique Sn/Pt est égal à 2,5.

Dans les exemples 1 à 3 le solvant comprend 15 $cm^3$ de benzène, le carbonate d'alcène étant respectivement:

le carbonate de propylène pour l'exemple 1,
le carbonate d'éthlène pour l'exemple 2, et
le carbonate d'hexylène-1,2 pour l'exemple 3

Dans l'exemple 4 le solvant comprend 2 $cm^3$ de benzène et 23 $cm^3$ de carbonate de propylène.

Exemples 5 à 8
Réaction d'hydroformylation

Un système catalytique préparé conformément à l'une des exemples 1 à 4 est introduit dans un réacteur autoclave de 100 $cm^3$ en acier ioxydable, équipé d'un système d'agitation par barreau aimanté, puis on ajoute 1,23 gramme de styrène. Le réacteur est chauffé jusqu'à la température de 80°C puis on charge le gaz de synthèse constitué d'un mélange équimolaire de monoxyde de carbone et d'hydrogène. Enfin le mélange est agité et la réaction est poursuivie à 80°C sous une pression de 50 bars pendant 24 heures. On obtient alors, avec un taux de transformation TT (experimé en pourcent), un mélange comprenant de l'éthylbenzène, du phényl-2 propanal et du phényl-3 propanal, L'analyse de ce mélange permet de déterminer d'une part la proportion en poids d'éthylbenzène EB (exprimée en pourcent) et d'autre part le rapport molaire n/b de l'aldéhyde normal à l'aldéhyde branché. Le tableau I ci-après résume les résultats obtenus en fonction du système catalytique utilisé.

TABLEAU I

| exemple | système catalyt. | TT | EB | n/b |
|---|---|---|---|---|
| 5 | ex. 1 | 40 | 2,5 | 6,25 |
| 6 | ex. 2 | 45 | 5 | 3,7 |
| 7 | ex. 3 | 38 | 12 | 2,3 |
| 8 | ex. 4 | 17 | 0,2 | 8,3 |

Exemple 9

Dans la cellule électrochimique décrite à l'exemple 1, on introduit le solvant constitué d'un mélange de 15 $cm^3$ de benzène et 10 $cm^3$ de carbonate de propylène, puis ou effectue la réduction électrochimique dudit solvant dans des conditions identiques à celles de l'exemple 1. On ajoute ensuite 60 mg de complexe $LPtCl_2$, le ligand L'étant le bis(diphénylphosphino)-1,4 butane.

Exemple 10

Le système catalytique préparé conformément à l'exemple 9 est utilisé pour effectuer la réaction d'hydroformylation de 3,57 gramme d'hexène-1 dans un réacteur autoclave indentique à celui de l'exemple 5, au moyen d'un mélange équimolaire d'hydrogène et de monoxyde de carbone. La pression P (exprimée en bars), la température T (exprimée en degrés Celsius) ainsi que la durée t (exprimée en heures) de la réaction sont indiquées au tableau II. On obtient alors, avec un taux de transformation TT (exprimée en pourcent), un mélange d'hexane, d'hexène-2, de méthyl-2 hexanal et de n-heptanal. L'analyse des aldéhydes formés permet de déterminer le rapport molaire n/b de l'adéhyde normal à l'aldéhyde branché. Le tableau II ci-après résume les résultats obtenus.

Exemples 11 à 13

On effectue la réduction électrochimique du solvant dans des conditions identiques à celles de l'exemple 9 à l'exception du potentiel de réduction ici égal à −60V. On ajoute ensuite 60 mg de complexe $LPtCl_2$, le ligand L'étant:

le (1SA,2S)(+)trans-1,2-bis(diphénylphosphinométhyl)-cyclohexane pour l'exemple 11.
l'isopropylidène dihydroxy-2,3-bis(diphénylphosphino)-1,4 butane pour 1'exmple 12.
le 1,4-bis(diphénylphosphino)butane pour l'exemple 13.

Exemples 14 et 15

Les systèmes catalytiques préparés conformément aux exemples 11 et 12 sont utilisés pour hydroformyler, l'hexène-1 dans les conditions de l'exemple 10. Le tableau II ci-après résume les résultats obtenus.

TABLEAU II

| exemple | système catalyt. | T | t | TT | EB | n/b |
|---|---|---|---|---|---|---|
| 10 | ex. 9 | 50 | 80 | 24 | 83 | 56 |
| 14 | ex. 11 | 100 | 90 | 6 | 100 | 28 |
| 15 | ex. 12 | 100 | 90 | 6 | 98 | 32 |

Examples 16 à 18

Les systémes catalytiques préparés conformément aux exemples 11 à 13 sont utilisés pour hydroformyler le styrène dans les conditions de l'exemple 5, aux exceptions suivants près:

quantité de styrène introduite dans le réacteur: 0,25 gramme.

pression de réaction: 100 bars.

La température T (exprimée en degrés Celsius) et la durée t (exprimée en heures) de la réaction sont indiquées au tableau III ci-après, ainsi que les résultats obtenus exprimés comme aux exemples 5 à 8 précédemment.

TABLEAU III

| exemple | système catalyt. | T | t | TT | EB | n/b |
|---|---|---|---|---|---|---|
| 16 | 11 | 90 | 4 | 100 | 7 | 3,8 |
| 17 | 12 | 100 | 24 | 67 | 8 | 3,0 |
| 18 | 13 | 100 | 24 | 25 | 4 | 3,0 |

Exemples 19 et 20

Dans une cellule électrochimique en verre on introduit sous azote un solvant constitué d'un mélange de 15 $cm^3$ de benzène et 10 $cm^3$ de carbonate de propylène. Dans ce solvant on plonge la cathode (constituée d'un panier de platine) et l'anode (constituée d'un cylindre d'étain). La température étant égale à 20°C, on fixe le potentiel de réduction à −60V. On observe la formation progressive d'un précipité que l'on isole par filtration et que l'on purifie par lavage. La poudre ainsi obtenue est soumise à une analyse élémentaire pondérale qui donne les résultats suivants:

| | |
|---|---|
| C | 14,0% |
| H | 1.8% |
| O | 20,6% |
| Sn | 63,6% |

Aprés dégazage du réacteur décrit à l'exemple 5, on introduit dans ce réacteur, sous balayage d'azote, le complexe $LPtCl_2$, le ligand L'étant:

le (1S,2S)(+)trans-1,2-bis(diphénylphosphinométhyl)-cyclohexane pour l'exemple 19.

L'isopropylidène dihydroxy-2,3-bis(diphénylphosphino)-1,4-butane pour l'exemple 20.

On introduit ensuite dans ce même réacteur, sous agitation, la poudre obtenue précédemment en quantité telle que le rapport atomique Sn/Pt soit égal à 1, puis le sytène en quantité telle que le rapport molaire du styrène au platine soit égal à 100. On chauffe ensuite l'autoclave à la température de 90°C après avoir chargé le gaz de synthèse, constitué d'un mélange équimolaire de monoxyde de carbone et d'hydrogène, à la pression de 100 bars. Le mélange est ensuite mis sous agitation. Après la durée de réaction t (exprimée en heures) spécifiée au tableau IV ci-après, le réacteur est refroidi puis le mélange gazeux éliminé. La phase liquide récupérée est analysée par chromatographie en phase gazeuse et les résultats obtenus, exprimés comme aux exemples 5 à 8 précédemment, sont indiqués au tableau IV.

TABLEAU IV

| exemple | t | TT | EB | n/b |
|---|---|---|---|---|
| 19 | 4 | 100 | 6 | 4,9 |
| 20 | 2,5 | 99 | 10 | 2,9 |

**Revendications**

1. Systéme catalytique caractérisé en ce qu'il comprend:

au moins un complexe de platine de formule $LPtX_2$ dans laquelle L est un composé organique comportant au moins deux atomes de phosphore capables de coordiner le platine et X est un atome d'halogène, et

au moins une combinaison complexe formée entre l'étain et un carbonate d'alcène, le groupe alcène ayant de préférence de 2 à 6 atomes de carbone.

2. Système catalytique selon la revendiction 1, caractérisé en ce que L est un bis(diphénylphosphino)alcane de formule

$$(C_6H_5)_2P\left(\begin{array}{c}R_1\\ |\\ C\\ |\\ R_2\end{array}\right)_n P(C_6H_5)_2 \qquad (I)$$

dans laquelle $R_1$ et $R_2$, identiques ou différents, sont choisis parmi l'atome d'hydrogène et les radicaux hydrocarbonés aliphatiques, ceux-ci étant le cas échéant fonctionnalisés et/ou liés l'un à l'autre, et n est supérieur ou égal à 4.

3. Système catalytique selon la revendication 1, caractérisé en ce que L est un aminophosphine-phosphinite de formule

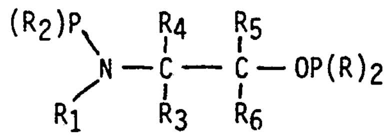

(II)

dans laquelle:

R est un radical hydrocarboné aliphatique, cycloaliphatique ou aromatique,

$R_1$ est choisi parmi l'atome d'hydrogène et les radicaux hydrocarbonés,

$R_3$ et $R_4$, nécessairement différents l'un de l'autre, sont choisis parmi l'atome d'hydrogène et les radicaux hydrocarbonés éventuellement porteurs d'au moins une fonction choisie parmi les fonctions alcool, thiol, thioether, amine, imine, acide, ester, amide et ether, et

$R_5$ et $R_6$ sont choisis parmi l'atome d'hydrogène et les radicaux hydrocarbonés éventuellement fonctionnalisés.

4. Système catalytique selon l'une des revendications 1 à 3, caractérisé en ce que le complexe de platine et la combinaison complexe à base d'étain sont présents en proportions respectives telles que le rapport atomique Sn/Pt soit compris entre 0,2 et 5.

5. Procédé de préparation d'un système catalytique selon la revendication 1, caractérisé en ce qu'on fait réagir au moins une combinaison complexe formée entre l'étain et le carbonate d'alcène avec au moins un complexe de platine de formule $LPtX_2$ dans au moins un solvant dudit complexe, à une température comprise entre 10°C et la température d'ébullition dudit solvant.

6. Procédé de préparation selon la revendication 5, caractérisé ce que la combinaison complexe d'étain et d'un carbonate d'alcène est formée par réduction d'un solvant électrochimique comportant au moins un carbonate d'alcène dans une cellule électrochimique comprenant une anode en étain et une cathode en platine en portant la cathode à un potentiel inférieur ou égal à −0,2 volt, et en maintenant constant ce potentiel pendant une durée suffisante pour assurer la production d'une quantité d'électricité supérieure ou égale à 0,5 Faraday par atome-gramme de platine.

7. Procédé selon la revendication 6, caractérisé en ce que la réduction du solvant électrochimique est effectuée en présence d'une faible quantité d'un sel conducteur soluble dans ledit solvant.

8. Procédé selon l'une des revendications 6 et 7, caractérisé en ce que la réduction du solvant électrochimique est effectuée à une température comprise entre 10°C et 70°C et en maintenant la cellule électrochimique sous atmosphère d'un gaz inerte.

9. Procédé selon l'une des revendications 6 à 8, caractérisé en ce que le solvant électrochimique comprend en outre un hydrocarbure aromatique en mélange avec le carbonate d'acène.

10. Procédé selon la revendication 5, caractérisé en ce que le solvant du complexe de platine est choisi parmi les hydrocarbures aromatiques et les carbonats d'alcène.

11. Procédé selon la revendication 10, caractérisé en ce que ledit solvant comprend au moins 25% en volume de carbonate d'alcène.

12. Procédé selon l'une des revendications 5 à 11, caractérisé en ce que la concentration du complexe de platine dans le solvant de la réaction est comprise entre 0,01 et 0,2 mole par litre.

13. Application d'un système catalytique selon la revendication 1 à la fabrication d'aldéhydes par hydroformylation d'un composé organique à insaturation éthylénique, caractérisé en ce que l'on fait réagir, à une température comprise entre 10° et 300°C et sous une pression comprise entre 1 et 350 bars, ledit composé organique avec un mélange de monoxyde de carbone et l'hydrogène en présence d'une quantité efficace dudit système catalytique.

14. Application selon la revendication 13, caractérisée en ce que le composé organique à insaturation éthylénique est choisi parmi les oléfines ayant de 2 à 12 atomes de carbone, les composés vinyliques aromatiques et les diènes.

**Patentansprüche**

1. Katalytisches System, dadurch gekennzeichnet, daß es mindestens einen Platinkomplex der allgemeinen Formel $LPtX_2$, in der L für eine organische Verbindung, welche mindestens zwei Phosphoratome, die imstande sind, das Platin kovalent zu binden, enthält, und X für ein Halogenatom stehen, und mindestens eine Komplexverbindung, welche zwischen Zinn und einem Alkencarbonat, wobei die Alkengruppe vorzugsweise 2 bis 6 Kohlenstoffatome enthält, gebildet wird, umfaßt.

2. Katalytisches System nach Anspruch 1, dadurch gekennzeichnet, daß L für ein bis(Diphenylphosphino)alkan der allgemeinen Formel:

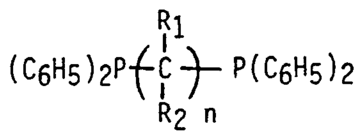

(I)

in welcher $R_1$ und $R_2$, welche gleich oder voneinander verschieden sind, unter dem Wasserstoffatom und den aliphatischen Kohlenwasserstoffgruppen, welche gegebenfalls Funktionen aufweisen und/oder miteinander verbunden sind, gewählt werden, steht, und n über oder gleich 4 ist.

3. Katalytisches System nach Anspruch 1, dadurch gekennzeichnet, daß L für einen Aminophosphin-phosphinit der allgemeinen Formel

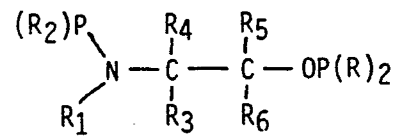

(II)

in welcher R eine aliphatische, cycloaliphatische oder aromatische Kohlenwasserstoffgruppe bedeutet, $R_1$ unter dem Wasserstoffatom und den Kohlenwasserstoffgruppen gewählt wird, $R_3$ und $R_4$, welche notwendigerweise voneinander verschieden sind, unter dem Wasserstoffatom und den Kohlenwasserstoffgruppen, welche gegebenenfalls mindestens eine Funktion, ausgewählt unter den Alkohol-, Thiol-, Thioether-, Amin-, Imin-, Säure-, Ester-, Amid- und Etherfunktionen, aufweisen, ausgewählt sind, und $R_5$ und $R_6$ unter dem Wasserstoffatom und den Kohlenwasserstoffgruppen, welche gegebenenfalls Funktionen aufweisen, ausgewählt sind.

4. Katalytisches System nach einem der Ansprüche 1—3, dadurch gekennzeichnet, daß der Platinkomplex und die komplexe Verbindung auf der Basis von Zinn in solchen Mengen vorhanden sind, daß das Atomverhältnis Sn/Pt zwischen 0,2 und 5 beträgt.

5. Verfahren zu Herstellung eines katalytischen System nach Anspruch 1, dadurch gekennzeichnet, daß man mindestens eine zwischen dem Zinn und dem Alkencarbonat gebildete Komplexverbindung mit mindestens einem Platinkomplex der allgemeinen Formel $LPtX_2$ in mindestens einem Lösungsmittel für diesen Komplex bei einer Temperatur zwischen 10°C und der Siedetemperatur dieses Lösungsmittels reagieren läßt.

6. Verfahren nach Anspruch 5, dadurch gekennzeichnet, daß die komplexe Verbindung von Zinn und einem Alkencarbonat durch Reduktion eines elektrochemischen Lösungsmittels, welches mindestens ein Alkencarbonat enthält, in einer elektrochemischen Zelle, welche eine Zinnanode und eine Platinkathode umfaßt, gebildet wird, wobei die Kathode auf ein Potential unter oder gleich −0,2 Volt gebracht und dieses Potential während eines Zeitraumes aufrechterhalten wird, welcher ausreicht, um die Erzeugung einer Elektrizitätsmenge über oder gleich 0,5 Faraday pro Grammatom Platin zu gewährleisten.

7. Verfahren nach Anspruch 6, dadurch gekennzeichnet, daß man die Reduktion des elektrochemischen Lösungsmittels in Gegenwart einer geringen Menge eines in diesem Lösungsmittel löslichen Leitsalzes durchführt.

8. Verfahren nach Anspruch 6 oder 7, dadurch gekennzeichnet, daß man die Reduktion des elektrochemischen Lösungsmittels bei einer Temperatur zwischen 10° und 70°C durchführt, wobei die elektrochemische Zelle unter einer Inertgas-atmosphäre gehalten wird.

9. Verfahren nach einem der Ansprüche 6—8, dadurch gekennzeichnet, daß man als das elektrochemische Lösungsmittel ein solches einsetzt, welches außerdem einen aromatischen Kohlenwasserstoff in Mischung mit dem Alkencarbonat enthält.

10. Verfahren nach Anspruch 5, dadurch gekennzeichnet, daß man als das Lösungsmittel des Platinkomplexes ein solches einsetzt, welches unter den aromatischen Kohlenwasserstoffen und den Alkencarbonaten ausgewahlt wird.

11. Verfahren nach Anspruch 10, dadurch gekennzeichnet, daß man als dieses Lösungsmittel ein solches ensetzt, welches mindestens 25 Vol.-% Alkencarbonat enthält.

12. Verfahren nach einem der Ansprüche 5—11, dadurch gekennzeichnet, daß man die Konzentration des Platinkomplexes in dem Reaktionslösungsmittel zwischen 0,01 und 0,2 Mol/Liter wählt.

13. Verwendung eines katalytischen Systems nach Anspruch 1 zur Herstellung von Aldehyden durch Hydroformylierung einer organischen Verbindung, welche eine ethylenische Ungesättigtheit aufweist, dadurch gekennzeichnet, daß man die organische Verbindung mit einem Gemisch von Kohlenmonoxid und Wasserstoff in Gegenwart einer wirksamen Menge des katalytischen Systems bei einer Temperatur zwischen 10° und 300°C und einem Druck zwischen 1 und 350 bar umsetzt.

14. Verwendung nach Anspruch 13, dadurch gekennzeichnet, daß die organische Verbindung mit ethylenischer Ungesättigtheit unter den Olefinen mit 2 bis 12 Kohlenstoffatomen, den aromatischen Vinylverbindungen und den Dienen ausgewählt ist.

**Claims**

1. Catalytic system characterized in that it comprises:

at least one platinum complex of formula $LPtX_2$ in which L is an organic compound comprising at least two phosphorus atoms which are capable of coordinating the platinum and X is a halogen atom, and

at least one complex combination formed between tin and an alkene carbonate, the alkene group preferably containing from 2 to 6 carbon atoms.

2. Catalytic system according to Claim 1, characterized in that L is a bis(diphenylphosphino)alkane of formula

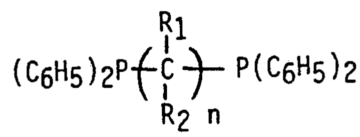

(I)

in which $R_1$ and $R_2$, which are identical or different, are chosen from the hydrogen atom and aliphatic hydrocarbon radicals, the latter bearing functional groups if appropriate, and/or being joined together, and n is greater than or equal to 4.

3. Catalytic system according to Claim 1, characterized in that L is an aminophosphinephosphinite of formula

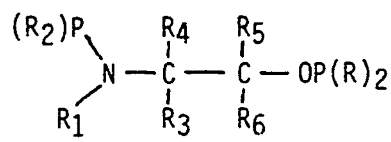

(II)

in which:

R is an aliphatic, alicyclic or aromatic hydrocarbon radical,

$R_1$ is chosen from the hydrogen atom and hydrocarbon radicals,

$R_3$ and $R_4$, which must differ from each other, are chosen from the hydrogen atom and hydrocarbon radicals which may, if appropriate, bear at least one functional group chosen from the alcohol, thiol, thioether, amine, imine, acid, ester, amide and ether groups, and $R_5$ and $R_6$ are chosen from the hydrogen atom and hydrocarbon radicals, bearing functional groups if appropriate.

4. Catalytic system according to one of Claims 1 to 3, characterized in that the platinum complex and the complex combination based on tin are present in respective proportions such that the atomic ratio Sn/Pt is between 0.2 and 5.

5. Process for the preparation of a catalytic system according to Claim 1, characterized in that at least one complex combination formed between tin and the alkene carbonate is reacted with at least one platinum complex of formula $LPtX_2$ in at least one solvent for the said complex, at a temperature of between 10°C and the boiling temperature of the said solvent.

6. Preparation process according to Claim 5, characterized in that the complex combination of tin and an alkene carbonate is formed by the reduction of an electrochemical solvent containing at least one alkene carbonate in an electrochemical cell comprising a tin anode and a platinum cathode by raising the cathode to a potential not exceeding −0.2 volt, and by maintaining this potential constant for a sufficient period to ensure the production of a quantity of electricity of at least 0.5 faraday per gram-atom of platinum.

7. Process according to Claim 6, characterized in that the reduction of the electrochemical solvent is carried out in the presence of a small quantity of conductive salt which is soluble in the said solvent.

8. Process according to either of Claims 6 or 7, characterized in that the reduction of the electrochemical solvent is carried out at a temperature of between 10°C and 70°C and while the electrochemical cell in maintained under an inert gas atmosphere.

9. Process according to one of Claims 6 to 8, characterized in that the electrochemical solvent additionally comprises an aromatic hydrocarbon mixed with the alkene carbonate.

10. Process according to Claim 5, characterized in that the solvent for the platinum complex is chosen from aromatic hydrocarbons and alkene carbonates.

11. Process according to Claim 10, characterized in that the said solvent comprises at least 25% by volume of alkene carbonate.

12. Process according to one of Claims 5 to 11, characterized in that the concentration of the platinum complex in the reaction solvent is between 0.01 and 0.2 mole per litre.

13. Application of a catalytic system according to Claim 1, to the production of aldehydes by the hydroformylation of an ethylenically unsaturated organic compound, characterized in that the said organic compound is reacted with a mixture of carbon monoxide and hydrogen, at a temperature of between 10° and 300°C and at a pressure of between 1 and 350 bars, in the presence of an effective quantity of the said catalytic system.

14. Application according to Claim 13, characterized in that the ethylenically unsaturated organic compound is chosen from olefins containing from 2 to 12 carbon atoms, aromatic vinyl compounds and dienes.